(19) Europäisches Patentamt
European Patent Office
Office européen des brevets

(11) **EP 2 930 301 B1**

(12) **FASCICULE DE BREVET EUROPEEN**

(45) Date de publication et mention
de la délivrance du brevet:
**17.07.2019 Bulletin 2019/29**

(51) Int Cl.:
***E21B 47/10*** *(2012.01)*      ***E21B 43/26*** *(2006.01)*

(21) Numéro de dépôt: **15305402.8**

(22) Date de dépôt: **19.03.2015**

(54) **PROCEDE DE SURVEILLANCE DE SITE D'EXPLORATION ET D'EXPLOITATION D'HYDROCARBURES NON CONVENTIONNELS**

ÜBERWACHUNGSVERFAHREN DES EXPLORATIONS- UND ABBAUORTS VON NICHT KONVENTIONELLEN KOHLENWASSERSTOFFEN

METHOD FOR MONITORING A SITE FOR EXPLORATION AND EXPLOITATION OF NON-CONVENTIONAL HYDROCARBONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.04.2014 FR 1453042**

(43) Date de publication de la demande:
**14.10.2015 Bulletin 2015/42**

(73) Titulaire: **IFP Energies nouvelles**
**92500 Rueil-Malmaison (FR)**

(72) Inventeur: **Garcia, Bruno**
**92500 Rueil Malmaison (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**EP-A1- 2 500 513      WO-A2-03/010534**

EP 2 930 301 B1

**Description**

La présente demande concerne le domaine de la récupération des hydrocarbures non conventionnels, et plus particulièrement la surveillance de site d'exploration et d'exploitation d'hydrocarbures non conventionnels.

Les hydrocarbures (liquides ou gazeux) non conventionnels désignent des ressources dont l'extraction nécessite un traitement de stimulation en plus des moyens classiques de forage, par opposition aux hydrocarbures conventionnels dont l'extraction ne nécessite pas de traitement spécifique si ce n'est l'injection d'eau et/ou de gaz pour augmenter le rendement de récupération d'hydrocarbures (récupération assistée d'hydrocarbures EOR). Les hydrocarbures non conventionnels se réfèrent donc à différents types de ressources, parmi lesquels :

- l'huile de schiste (shale oil) est une huile piégée dans la roche-mère suite à son enfouissement profond ;
- l'huile de réservoir compact (tight oil) est une huile parvenue à migrer depuis la roche-mère, mais qui se trouve localisée dans un réservoir très peu perméable;
- le gaz de réservoir compact ou gaz compact (tight gaz) est le gaz qui s'est accumulé dans un réservoir peu perméable, comme l'huile évoquée ci-dessus. Il s'agit généralement de méthane en position intermédiaire entre le gaz de schiste et le gaz conventionnel ;
- le gaz de houille (coalbed methane-CBM) est un gaz qui se rencontre dans les couches de charbon, riches en méthane adsorbé, que les mineurs nomment « grisou ». Il est produit par de simples forages verticaux quand une fracturation naturelle de la roche a suffi à libérer une quantité suffisante de méthane pour générer un débit significatif. Dans le cas contraire, il faut stimuler la roche par fracturation hydraulique ;
- le gaz de mine (coal mine methane-CMM) est un gaz de la même nature que le précédent, que l'on récupère par simple pompage dans les anciennes mines non ennoyées, notamment en France dans les anciens filons du Nord-Pas-de-Calais ;
- les schistes bitumineux (oil shale) et les sables bitumineux (oil sands) sont des matières organiques qui ne sont pas restées suffisamment longtemps dans la roche-mère pour se transformer en hydrocarbures. Leur exploitation ne nécessite pas de fracturation hydraulique mais un traitement thermique extrêmement coûteux en énergie ;
- le gaz de schiste (shale gaz) est un gaz demeuré emprisonné dans la roche sédimentaire, situé à une profondeur de 2000 à 3000 mètres de la surface. Le méthane y est contenu dans des micropores ne communiquant pas, il est éventuellement adsorbé sur des particules argileuses imperméables. Le milieu tient donc à la fois de la roche mère et du réservoir, mais cette imperméabilité empêche son extraction par des moyens classiques de forage.

L'extraction des hydrocarbures non conventionnels, particulièrement difficile, nécessite le recours systématique aux techniques combinées du forage dirigé et de la fracturation, particulièrement couteuses. Par exemple, l'exploitation d'hydrocarbures non conventionnels tels que les hydrocarbures de roches mères (Shale-Plays) : huile de schiste (Oil-Shale) et gaz de schiste (Gas-Shale) nécessite une fracturation de la roche encaissante pour la libération de ces hydrocarbures. En effet, contrairement au gaz naturel conventionnel qui est retenu dans une roche perméable permettant une exploitation facile, le gaz de schiste est piégé dans les porosités d'une roche rendue imperméable par l'argile qu'elle contient.

Pour l'ensemble des hydrocarbures de roches mères, hydrocarbures non conventionnels cités ci-dessus ; on parle de roches situées aux alentours de 2000 à 4000 m de profondeur. Ces roches sont naturellement fracturées ou fissurées, mais les fractures ou fissures sont fermées, ce qui ne permet donc pas à un fluide de circuler et de passer à travers. L'objectif de la fracturation est de ré-ouvrir et d'étendre ce réseau de fractures/fissures. Pour cela, une des méthodes de fracturations possible est l'injection sous pression d'un liquide. Mais à ces profondeurs, les fractures vont naturellement se refermer, il faut donc étayer ce réseau de fractures, et faire en sorte qu'il reste ouvert pour que le gaz et/ou l'huile puisse circuler vers un puits producteur pour être extrait vers la surface.

Plusieurs types de fracturation existent telles que :

- la fracturation atomique (1967) qui a été abandonnée mais qui consistait à faire exploser dans le sous-sol une bombe atomique pour créer un réseau de fractures/fissures afin de pouvoir exploiter ces hydrocarbures
- la fracturation hydraulique, qui est une technique qui permet de ré-ouvrir un réseau de fissures qui existent déjà naturellement, de l'étendre et donc de créer un plus large réseau de fissures. Avec l'eau de fracturation, on rajoute des particules (propant), des micro-billes de silice (du sable), qui vont venir tapisser les parois de la fracture/fissure, permettant aux fractures/fissures créées de perdurer et de ne pas se refermer naturellement. On rajoute aussi un viscosifiant, un bactéricide (pour tuer les bactéries éventuellement présentes) et un produit type polymère pour que le fluide chargé en particules circule plus facilement en profondeur et permettant par la même une diminution de la consommation d'énergie en surface. On utilise également des additifs issus de l'agroalimentaire et des produits biodégradables. La fracturation hydraulique nécessite une quantité d'eau relativement importante : 10 000 à 20 000 m$^3$ d'eau par forage.

- la fracturation mécanique par explosion (dynamite), ou encore
- la fracturation par injection de gaz tel que le fluoropropane (propane non inflammable), et l'hélium à chaud.

L'exploitation à grande échelle des hydrocarbures non conventionnels a démarré au cours des années 2000 lorsque le prix des hydrocarbures s'est établi durablement au-dessus d'un seuil élevé en relation avec la stagnation de la production du pétrole et du gaz conventionnel et la croissance de la consommation énergétique mondiale. Ces prix ainsi que les avancées dans le domaine des techniques d'extraction ont permis de financer les investissements très importants nécessaires pour permettre la mise en production de nombreux puits, notamment aux États-Unis. 600 000 emplois ont été créés aux Etats-Unis avec l'exploitation de ce type d'hydrocarbures. Le prix du charbon aux USA a baissé de 30 à 40%, le prix du gaz a également baissé aux USA mais pas encore dans le monde. De plus, le prix du pétrole est impacté parce que les USA en importent moins.

Un risque souvent évoqué par rapport à l'exploration et l'exploitation de ces hydrocarbures non conventionnels concerne le fait que l'eau de fracturation (lors de la fracturation hydraulique notamment) puisse entrer en conflit avec des eaux d'autres usages, type eaux potables. Or, il est tout à fait envisageable d'utiliser de l'eau impropre à la consommation pour la fracturation hydraulique telle que l'eau de mer (même si cela engendre des contraintes type sulfates, $H2S...$,.mais on sait s'en accommoder), ou encore des eaux saumâtres (saumures salines).

Un autre risque important souvent évoqué est celui de la contamination directe de la fracturation hydraulique avec des eaux potables naturelles présentes dans le sous-sol. Mais vu les profondeurs auxquelles cette fracturation hydraulique est réalisée (pour rappel entre 2000 et 4000 m de profondeur), le risque de contamination directe est très difficilement envisageable. Par contre, un risque plus important existe le long du puits dû à l'étanchéité de celui-ci.

Ainsi, les problèmes environnementaux associés à l'extraction du gaz de schiste, notamment la pollution des réserves d'eau liée aux fuites potentielles, notamment au sein d'un aquifère d'eau potable (même si celui-ci est quasi nul), d'hydrocarbures et de fluides injectés pour la fracturation dans le sous-sol, ainsi que l'émission de gaz à effet de serre, entraînent dans certains pays, une défiance importante de l'opinion publique.

Par ailleurs, on connaît différentes méthodes de surveillance de réservoir souterrain adaptées au domaine du stockage géologique de gaz. Les demandes de brevet EP 2500513, qui est considéré l'état de la technique le plus proche, 30 et WO 03010534 décrivent de telles méthodes.

Afin de limiter ces problèmes environnementaux, la surveillance des sites de récupération d'hydrocarbures non conventionnels au cours de l'exploration, puis pendant et après l'exploitation, c'est-à-dire l'extraction des hydrocarbures non conventionnels, est un enjeu important pour le développement de cette technologie.

Pour cette surveillance, on peut envisager d'injecter dans la formation souterraine un gaz traceur dont l'inertie chimique est totale. Cependant cette méthode nécessitant l'injection d'un gaz particulier en grande quantité vu les volumes impactés est complexe à mettre en oeuvre.

L'objet de la présente demande concerne un procédé de surveillance d'un site de récupération d'hydrocarbures non conventionnels permettant de quantifier les hydrocarbures présents dans les zones au-dessus de la zone d'exploration et d'exploitation. Le procédé selon la demande se fonde sur l'ajustement d'un modèle décrivant la concentration en gaz en fonction du temps, au moyen d'analyses géochimiques in-situ de gaz rares et le cas échéant de gaz injecté utilisé pour la fracturation, contenus dans des phases fluides d'échantillons du sous-sol. Grâce à l'analyse des gaz rares, le procédé selon la demande permet d'anticiper une fuite d'hydrocarbures au-dessus du site d'exploration/d'exploitation.

**Le procédé selon la demande** La demande concerne un procédé de surveillance d'une formation souterraine, dans laquelle on récupère des hydrocarbures non conventionnels, au moins un gaz rare étant présent dans ladite formation souterraine. Pour ce procédé, on réalise les étapes suivantes :

a) on choisit un modèle de diffusion dudit gaz rare et un modèle de diffusion d'un hydrocarbure à récupérer, chaque modèle décrivant l'évolution de la concentration en fonction du temps, de la profondeur et d'un coefficient de diffusion ;
b) avant ladite récupération d'hydrocarbures, on prélève au moins un premier échantillon d'un fluide présent dans une zone dite de surveillance du sous-sol située au-dessus de ladite formation souterraine, et on mesure la composition d'au moins un gaz rare au sein dudit premier échantillon ;
c) pendant et/ou après ladite récupération d'hydrocarbures, on prélève au moins un second échantillon d'un fluide présent dans ladite zone de surveillance, et on mesure une concentration dudit gaz rare au sein dudit second échantillon ;
d) on répète l'étape c) à différents instants ;
e) lorsque ladite concentration dudit gaz rare augmente, on modifie ledit coefficient de diffusion effective du modèle de diffusion du gaz rare, de façon à ce que le modèle de diffusion du gaz rare soit cohérent avec lesdites mesures, et on déduit un ratio entre ledit coefficient de diffusion et ledit coefficient de diffusion modifié ; et
f) on applique ledit ratio au modèle de diffusion dudit hydrocarbure à récupérer, à partir duquel on détermine la quantité dudit hydrocarbure à récupérer présent dans ladite zone de surveillance à l'instant t.

Selon la demande, ladite zone de surveillance est un aquifère.

Avantageusement, on détecte une fuite dudit hydrocarbure à récupérer hors de la formation souterraine au moyen de ladite quantité déterminée d'hydrocarbure à récupérer présent dans ladite zone de surveillance.

De préférence, on récupère lesdits hydrocarbures non conventionnels au moyen d'une fracturation de ladite formation souterraine.

De manière avantageuse, ladite fracturation est réalisée par injection de gaz, tel que le fluoropropane ou l'hélium.

De manière préférentielle, on détermine la quantité dudit gaz injecté présent dans ladite zone de surveillance à l'instant t au moyen d'un modèle de diffusion dudit gaz injecté, auquel on applique ledit ratio entre ledit coefficient de diffusion et ledit coefficient de diffusion modifié.

Selon l'invention, ledit gaz injecté comporte au moins un gaz rare.

Selon un mode de réalisation de l'invention, les hydrocarbures comprennent du méthane.

Avantageusement, ledit gaz rare est de l'hélium ou de l'argon.

## Présentation succincte des figures

D'autres caractéristiques et avantages du procédé selon la demande, apparaîtront à la lecture de la description ci-après d'exemples non limitatifs de réalisations, en se référant aux figures annexées et décrites ci-après.

La figure 1 illustre un exemple de récupération d'hydrocarbures non conventionnels.
La figure 2 illustre des modèles de diffusion de l'hélium (He), de l'argon (Ar), du fluoropropane (FP) et du méthane ($CH_4$) calés au moyen de mesures géochimiques.
La figure 3 illustre l'amélioration du calage avec des mesures géochimiques ultérieures.
La figure 4 illustre le calcul du délai dont on dispose pour agir et remédier à une future fuite du $CH_4$.

## Description détaillée de la demande

Le procédé de surveillance selon la demande concerne un site d'exploration ou d'exploitation des hydrocarbures non conventionnels.

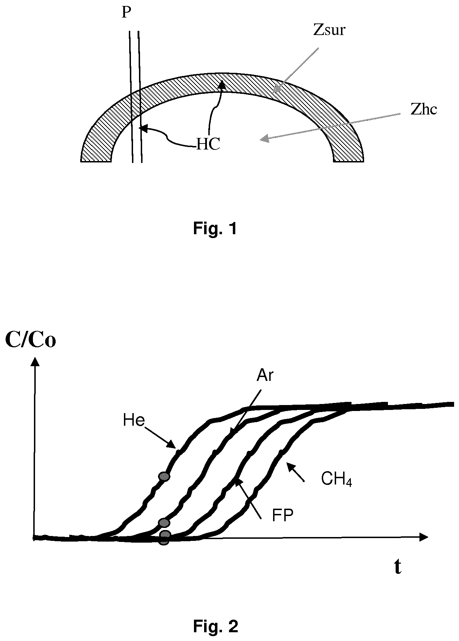

Selon un exemple non limitatif de réalisation illustré à la figure 1, la récupération des hydrocarbures non conventionnels HC, contenus dans une formation souterraine Zhc, sous forme de roche encaissante particulière, par exemple une roche de type argileuse à très faible perméabilité, est réalisée au moyen d'un puits P. La zone contenant les hydrocarbures Zhc est couverte par une zone supérieure, notamment un aquifère contenant de l'eau. La récupération des hydrocarbures non conventionnels HC nécessite un traitement spécifique, notamment une fracturation de la formation souterraine Zhc. Cette fracturation permet la récupération des HC au moyen du puits P, mais peut engendrer une migration des hydrocarbures dans la zone supérieure. Cette migration peut être, soit advective si la fracturation s'est révélée trop importante et mal contrôlée, soit diffusive ce qui est le cas pour tous les sites. La diffusion étant indépendante de la perméabilité et seulement dépendante de la porosité, même dans le cas d'une couche à très faible perméabilité, le phénomène de diffusion aura lieu. Dans le cas de la diffusion, sur la figure 1, la diffusion des hydrocarbures HC (méthane $CH_4$, éthane $C_2H_6$, propane $C_3H_8$, butane $C_4H_{10}$...) est symbolisée par des flèches courbes. Le méthane étant l'espèce majoritaire dans la plupart des cas et étant l'espèce qui diffuse le plus vite parmi les hydrocarbures car sa molécule est la plus petite. Une diffusion dans la zone supérieure, plus particulièrement s'il s'agit d'un aquifère est à éviter afin de limiter les problèmes environnementaux liés à l'exploitation des hydrocarbures non conventionnels. Le procédé selon la demande permet de surveiller cette diffusion dans cette zone supérieure, appelée zone de surveillance Zsur. Avantageusement, cette zone de surveillance Zsur correspond à un aquifère salin, constituée d'eau non potable, avant d'avoir un aquifère d'eau potable (plus haut (proche de la surface) dans la colonne d'eau).

Plusieurs types de fracturation existent telles que (i) l'injection d'eau sous pression (on parle alors de fracturation hydraulique), par exemple associée à des micro-billes de silice (sable) pour garder la fracturation créée ouverte, telles que (ii) la fracturation mécanique par explosion (dynamite) ou encore (iii) par injection de gaz tel que le fluoropropane (propane non inflammable) ou encore de l'hélium chaud (notamment pour les forages/fracturations en région arctique où l'eau gèle trop rapidement et où le fluoropropane ne semble pas efficace pour les mêmes raisons).

Le gaz injecté, par exemple le fluoropropane ou l'hélium, utilisé comme « agent de fracturation », peut également fuir par diffusion et polluer la zone sus-jacente à la roche fracturée et exploitée. Cette diffusion peut être également évitée afin de limiter les problèmes environnementaux liés à l'exploitation des hydrocarbures non conventionnels par fracturation par injection de gaz.

Le procédé de surveillance selon la demande permet de quantifier les hydrocarbures diffusés dans la zone supérieure et permet d'anticiper une fuite d'hydrocarbures dans cette zone au moyen d'une analyse des gaz rares notamment et éventuellement du gaz injecté pour fracturer (par exemple le fluoropropane ou l'hélium) présents dans la zone supérieure. En effet, la formation souterraine dans laquelle se trouvent les hydrocarbures non conventionnels contient également des gaz rares (par exemple de l'hélium ou de l'argon) qui vont également diffuser vers la zone supérieure. L'hélium est naturellement présent dans les environnements géologiques, d'autant plus s'ils sont profonds. De plus, pour la fracturation par injection de gaz, le gaz injecté peut aussi comprendre des gaz rares qui vont diffuser vers la zone supérieure (notamment dans le cas d'injection d'hélium chaud).

Le procédé de surveillance se base sur l'utilisation de trois caractéristiques intéressantes des gaz rares par rapport aux hydrocarbures et le cas échéant au gaz injecté :

- une diffusion plus rapide en milieu aqueux ;

- une détectabilité par les outils de mesure plus fine ; et

- une inactivité avec leur environnement d'un point de vue chimique et biologique.

Le procédé de surveillance comporte essentiellement les étapes suivantes :

   1. Choix de modèles de diffusion d'un gaz rare et des hydrocarbures ;

   2. Mesure de concentration en gaz rare, avant exploration et exploitation ;

   3. Mesures de concentration en gaz rare pendant l'exploration, pendant et après exploitation ;

   4. Calage du modèle de diffusion du gaz rare avec les mesures de concentrations ;

   5. Mise à jour de modèle de diffusion des hydrocarbures, à partir du modèle de diffusion du gaz rare calé ;

   6. Détermination de la quantité des hydrocarbures présents dans la zone de surveillance à un instant t à partir du modèle mis à jour.

Ces étapes sont détaillées ci-après pour un exemple non limitatif pour lequel le méthane $CH_4$ est un hydrocarbure à récupérer contenu dans la formation souterraine. Toutefois, ces étapes sont adaptées à tout type d'hydrocarbures contenus dans la formation souterraine, par exemple l'éthane $C_2H_6$, le propane $C_3H_8$, le butane $C_4H_1$, ou leurs mélanges.

1. Choix de modèles de diffusion d'un gaz rare et du $CH_4$ ;

On choisit un modèle de diffusion du gaz rare, par exemple l'hélium qui est naturellement présent dans les environnements géologiques, et un modèle de diffusion d'un hydrocarbure, le $CH_4$. Chaque modèle décrit l'évolution de la concentration de l'espèce chimique en fonction du temps, de la profondeur et d'un coefficient de diffusion propre à l'hélium et au $CH_4$.

On connaît par exemple un modèle 1D vertical de migration d'un constituant par diffusion, où l'évolution de la concentration (C) du constituant dans l'espace et le temps (t) est défini par :

$$C(z,t) = C_0 \, erfc\left(\frac{z}{2\sqrt{Dt}}\right) \qquad \text{Équation 1}$$

avec :

- $z$: profondeur
- $t$: temps
- D : coefficient de diffusion effective du constituant (gaz rare, hydrocarbures, gaz injecté) tel que D=Dm*ratio, où Dm est le coefficient de diffusion moléculaire du constituant et ratio (initialement égal à la porosité) un paramètre à mettre à jour lors de l'étape 4 du procédé ;
- $C_0$ : concentration maximale du constituant dissous (He ou $CH_4$), c'est-à-dire la concentration initiale avant injection. Il s'agît de la concentration située à l'interface eau/gaz.

Selon un mode de réalisation de la demande, selon lequel la récupération des hydrocarbures est mise en oeuvre par fracturation par injection de gaz, on choisit de la même manière un modèle de diffusion du gaz injecté, par exemple un modèle 1D tel que décrit à l'équation 1.

La figure 2 illustre des modèles (courbes) de diffusion de l'hélium (He), de l'argon (Ar), du fluoropropane (FP) (gaz injecté pour la fracturation) et du $CH_4$ (hydrocarbures).

2. Mesure de concentration en gaz rare avant exploration/exploitation

Avant l'exploitation et avant l'exploration (si l'on considère que l'exploration se comporte une étape de fracturation), c'est-à-dire avant la fracturation et la récupération des hydrocarbures, on prélève au moins un premier échantillon d'un fluide présent dans une zone de surveillance (zone du sous-sol susceptible d'être atteinte par les hydrocarbures), et on mesure la concentration du gaz rare au sein de ce premier échantillon. Pour réaliser ce prélèvement, on utilise au moins un puits de surveillance dans lequel on immerge un préleveur de fond permettant de récupérer le fluide (eau) en présence, sans perturber l'équilibre physico-chimique du système.

Ce puits permet de placer le préleveur dans une zone du sous-sol susceptible d'être atteinte par les hydrocarbures. Cette zone de surveillance est une zone située au-dessus de la formation souterraine contenant les hydrocarbures, il peut s'agir notamment d'un aquifère.

On réalise ensuite sur ce premier prélèvement une première mesure, qui caractérise l'état initial de la zone du sous-sol avant exploitation. On mesure la concentration du gaz rare au sein de ce premier échantillon. On peut également mesurer les concentrations d'autres gaz rares, des hydrocarbures, et du gaz injecté si cela est possible.

Cette étape permet de contraindre les compositions des hydrocarbures (éléments sous forme gaz et dissous dans la formation souterraine). La détection précoce de l'hélium par rapport au méthane dépend de la différence de composition en hélium dans la formation contenant les hydrocarbures par rapport à la zone de surveillance. L'hélium étant naturellement présent dans les environnements géologiques, d'autant plus s'ils sont profonds, cette différence doit être bien contrainte par l'analyse préliminaire des fluides naturels.

3. Mesures de concentration en gaz rare pendant et après exploitation

Pendant et après la fracturation et l'exploitation (récupération des hydrocarbures), on prélève au moins un second échantillon d'un fluide présent dans la zone de surveillance, et on mesure la concentration du gaz rare au sein de ce second échantillon.

Pour ce faire on utilise les mêmes moyens que pour l'étape précédente : puits de surveillance et préleveur de fond.

Cette étape peut être répétée à différents instants, et éventuellement au niveau de différents puits de surveillance.

On obtient ainsi un ensemble de valeurs du rapport de la concentration en gaz rare à un instant $t$ pendant et après l'exploitation, sur la concentration en gaz rare avant exploitation (un à chaque instant $t$ de prélèvement et mesure).

4. Calage du modèle de diffusion du gaz rare avec les mesures de concentrations

Lorsque la concentration du gaz rare (He) augmente, on modifie alors le coefficient de diffusion effective du modèle choisi à l'étape 1, de façon à ce que ce modèle soit cohérent avec les mesures. On en déduit alors le ratio entre le coefficient de diffusion moléculaire et le coefficient de diffusion modifié (Equation 1).

On prend l'hypothèse simple que le ratio est caractéristique du milieu (porosité et tortuosité) uniquement. Si l'on utilise plusieurs gaz rares, on calcule un ratio moyen modulo une erreur.

La figure 2 illustre cette étape de calage de la solution analytique (Equation 1) caractérisant l'évolution de la concentration du gaz rare en fonction du temps et de la position du point de mesure, avec les mesures réalisées à l'étape 3. Les points de mesure sont illustrés par des points sur la figure 2. La figure 2 illustre des modèles (courbes) de diffusion de l'hélium (He) et de l'argon (Ar) obtenus en calant la solution analytique (Equation 1) sur les points de mesures. Ces deux courbes calées permettent de définir un ratio moyen entre la diffusion moléculaire et la diffusion effective. Ce ratio permet de caler le modèle (courbes) de diffusion du $CH_4$ et du gaz injecté, le fluoropropane (FP) par exemple.

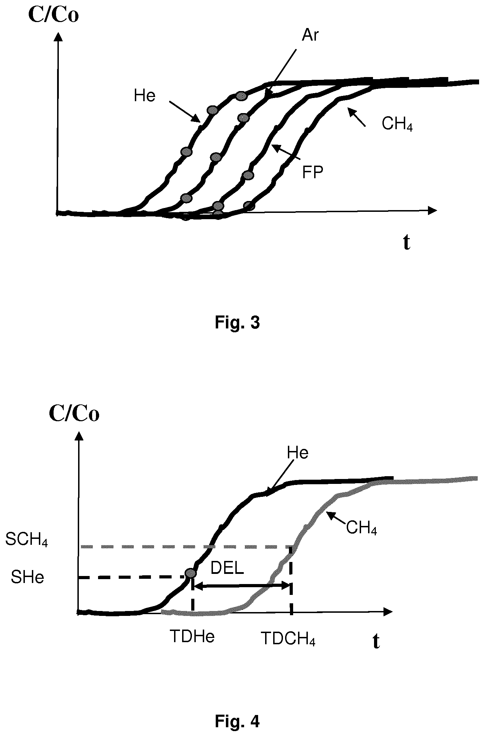

Les mesures effectuées à des dates ultérieures permettent d'affiner les résultats en modifiant le ratio (figure 3).

5. Mise à jour de modèle de diffusion des hydrocarbures et du gaz injecté

Pour mettre à jour le modèle de diffusion du $CH_4$ à partir du modèle de diffusion du gaz rare calé, on applique le ratio calculé à l'étape précédente au modèle de diffusion du $CH_4$ choisi à l'étape 1.

Pour ce faire, on applique ce ratio à la diffusion moléculaire du $CH_4$ utilisée dans le modèle de diffusion du $CH_4$ choisi à l'étape 1. On obtient une nouvelle diffusion effective du $CH_4$ qui permet d'obtenir un nouveau modèle de

diffusion du $CH_4$ basé sur le modèle de diffusion du gaz rare qui a été calé sur des données expérimentales à l'étape 4.

Pour le mode de réalisation de la demande, selon lequel la récupération des hydrocarbures est mise en oeuvre par fracturation par injection de gaz, on peut mettre à jour le modèle de diffusion du gaz injecté en appliquant le même ratio, ce qui permet d'obtenir un nouveau modèle de diffusion du gaz injecté basé sur le modèle de diffusion du gaz rare qui a été calé sur données expérimentales.

Cette étape est illustrée sur les figures 2 et 3.

6. Détermination de la quantité des hydrocarbures dans la zone de surveillance à partir du modèle mis à jour

Lors de cette étape, on détermine à chaque instant t la quantité des hydrocarbures présents dans la zone de surveillance, afin de déterminer une fuite d'hydrocarbures dans la zone de surveillance. Pour cela, on peut mettre en oeuvre les étapes suivantes :

a) détermination de la quantité de $CH_4$ dissout ; et
b) détermination d'une fuite de $CH_4$.

*a) Détermination de la quantité de $CH_4$ dissout à un instant t à partir du modèle mis à jour*

On utilise ensuite le modèle de diffusion du $CH_4$ ainsi mis à jour, pour déterminer la quantité de $CH_4$ dissout à un instant t.

Par intégration volumique du modèle (équation 1 mise à jour), on en déduit la masse de $CH_4$ dissout à un instant t :

$$M(t) = 2\phi SMC_0 \sqrt{\frac{Dt}{\pi}} \qquad\qquad \text{Équation 2}$$

avec :

$\phi$     : porosité du milieu ;
S     : surface de contact eau/gaz ;
M     : masse molaire du $CH_4$.

*b) Détermination d'une fuite de $CH_4$ vers un aquifère supérieur*

Selon un mode de réalisation, il est également possible de déterminer une fuite du $CH_4$ injecté en dehors de la zone de stockage (réservoir). Selon ce procédé, la zone de surveillance, c'est-à-dire la zone dans laquelle on prélève des échantillons au moyen d'un puits de surveillance et d'un préleveur, est un aquifère situé au-dessus de la zone du sous-sol dans lequel le $CH_4$ est injecté. On détecte alors une fuite de $CH_4$ hors de la zone d'injection en déterminant la quantité de $CH_4$ dissout dans cet aquifère (au moyen du modèle de diffusion du $CH_4$ (étape 6a) de l'invention).

Ce type de surveillance, au niveau d'un aquifère supérieur permet d'éviter la mise en place d'un puits de surveillance à travers la couverture géologique qui maintient le $CH_4$ dans la formation souterraine. De plus, le phénomène de diffusion est de loin le phénomène prépondérant au sein de la roche couverture, ce qui est d'autant plus intéressant vis-à-vis des gaz rares, et donc du procédé selon la demande.

Ce procédé permet de quantifier le délai que l'on dispose avant que l'on puisse détecter une fuite de $CH_4$ par des moyens classiques, et mettre en place des protocoles de remédiation et de colmatage de fuite. Il est basé sur le fait que les gaz rares ont un seuil de détection beaucoup plus bas que le $CH_4$ et qu'ils diffusent plus vite. Le procédé selon la demande détecte ainsi une fuite de $CH_4$ avant qu'une augmentation de concentration en $CH_4$ dans l'aquifère ne soit détectable par une mesure géochimique.

La figure 4 illustre les modèles (courbes) de diffusion de l'hélium et du $CH_4$ calés sur des mesures. SHe indique le seuil de détection par mesure géochimique d'une augmentation de la concentration en hélium. $SCH_4$ indique le seuil de détection par mesure géochimique d'une augmentation de la concentration en $CH_4$. Ainsi, TDHe indique la date à laquelle une augmentation de concentration en hélium est détectable, et $TDCH_4$ indique la date à laquelle une augmentation de concentration en $CH_4$ est détectable. On peut alors calculer le délai DEL dont on dispose pour agir et remédier à une potentielle fuite de $CH_4$.

Pour le mode de réalisation de la demande, selon lequel la récupération des hydrocarbures est mise en oeuvre par fracturation par injection de gaz, on peut déterminer la quantité de gaz injecté dans la zone de surveillance, afin de détecter une fuite du gaz injecté dans la zone de surveillance. Cette détermination peut être réalisée au moyen du modèle de diffusion du gaz injecté mis à jour, en effectuant de la même manière les étapes a), b), et c) décrites ci-dessus.

Et grâce à l'analyse du gaz injecté utilisé pour la fracturation (et ce à très grand volume), le procédé selon l'invention permet d'anticiper une fuite d'hydrocarbures au-dessus du site d'exploration/d'exploitation.

La fracturation par injection d'hélium est avantageuse pour le procédé selon la demande, car l'hélium (gaz rare) est injecté pour fracturer la roche en grande quantité, et ainsi, en cas de fuite, l'hélium est présent en plus grande quantité dans la zone de surveillance, ce qui permet une détection plus facile et plus rapide de la fuite.

Le procédé selon la demande est particulièrement adapté à la surveillance d'une formation souterraine de laquelle on extrait de l'huile de schiste et/ou du gaz de schiste, et pour laquelle on réalise une fracturation par injection de gaz tel que le fluoropropane ou l'hélium.

## Revendications

1. Procédé de récupération des hydrocarbures non conventionnels au sein d'une formation souterraine, dans lequel on récupère lesdits hydrocarbures non conventionnels au moyen d'une fracturation de ladite formation souterraine, au moins un gaz rare étant présent dans ladite formation souterraine, dans lequel on surveille ladite formation souterraine en réalisant les étapes suivantes :

   a) on choisit un modèle de diffusion dudit gaz rare et un modèle de diffusion d'un hydrocarbure à récupérer, chaque modèle décrivant l'évolution de la concentration en fonction du temps, de la profondeur et d'un coefficient de diffusion ;
   b) avant ladite récupération d'hydrocarbures, on prélève au moins un premier échantillon d'un fluide présent dans une zone dite de surveillance du sous-sol située au-dessus de ladite formation souterraine, et on mesure la composition d'au moins un gaz rare au sein dudit premier échantillon ;
   c) pendant et/ou après ladite récupération d'hydrocarbures, on prélève au moins un second échantillon d'un fluide présent dans ladite zone de surveillance, et on mesure une concentration dudit gaz rare au sein dudit second échantillon ;
   d) on répète l'étape c) à différents instants ;
   e) lorsque ladite concentration dudit gaz rare augmente, on modifie ledit coefficient de diffusion effective du modèle de diffusion du gaz rare, de façon à ce que le modèle de diffusion du gaz rare soit cohérent avec lesdites mesures, et on déduit un ratio entre ledit coefficient de diffusion et ledit coefficient de diffusion modifié ; et
   f) on applique ledit ratio au modèle de diffusion dudit hydrocarbure à récupérer, à partir duquel on détermine la quantité dudit hydrocarbure à récupérer présent dans ladite zone de surveillance à l'instant t.

2. Procédé selon la revendication 1, dans lequel ladite zone de surveillance est un aquifère.

3. Procédé selon l'une des revendications précédentes, dans lequel on détecte une fuite dudit hydrocarbure à récupérer hors de la formation souterraine au moyen de ladite quantité déterminée d'hydrocarbure à récupérer présent dans ladite zone de surveillance.

4. Procédé selon l'une des revendications précédentes, dans lequel ladite fracturation est réalisée par injection de gaz, tel que le fluoropropane ou l'hélium.

5. Procédé selon la revendication 4, dans lequel on détermine la quantité dudit gaz injecté présent dans ladite zone de surveillance à l'instant t au moyen d'un modèle de diffusion dudit gaz injecté, auquel on applique ledit ratio entre ledit coefficient de diffusion et ledit coefficient de diffusion modifié.

6. Procédé selon l'une des revendications précédentes, dans lequel ledit gaz injecté comporte au moins un gaz rare.

7. Procédé selon l'une des revendications précédentes, dans lequel les hydrocarbures comprennent du méthane.

8. Procédé selon l'une des revendications précédentes, dans lequel ledit gaz rare est de l'hélium ou de l'argon.

## Patentansprüche

1. Verfahren zur Wiedergewinnung der nicht konventionellen Kohlenwasserstoffe innerhalb einer unterirdischen Formation, bei dem die nicht konventionellen Kohlenwasserstoffe mit Hilfe einer Frakturierung der unterirdischen Formation wiedergewonnen werden, wobei mindestens ein Edelgas in der unterirdischen Formation vorhanden ist, bei dem die unterirdische Formation überwacht wird, wobei die folgenden Schritte durchgeführt werden:

a) Auswahl eines Diffusionsmodells des Edelgases und eines Diffusionsmodells eines wiederzugewinnenden Kohlenwasserstoffes, wobei jedes Modell die Entwicklung der Konzentration in Abhängigkeit von der Zeit, der Tiefe und einem Diffusionskoeffizienten beschreibt;

b) vor der Wiedergewinnung von Kohlenwasserstoffen Entnahme mindestens einer ersten Probe eines Fluids, das in einer so genannten Überwachungszone des Unterbodens, der sich über der unterirdischen Formation befindet, vorhanden ist, und Messen der Zusammensetzung mindestens eines Edelgases in der ersten Probe;

c) während und/oder nach der Wiedergewinnung von Kohlenwasserstoffen Entnahme mindestens einer zweiten Probe eines in der Überwachungszone vorhandenen Fluids und Messen einer Konzentration des Edelgases in der zweiten Probe;

d) Wiederholen des Schritts c) zu verschiedenen Zeitpunkten;

e) wenn die Konzentration des Edelgases steigt, Verändern des effektiven Diffusionskoeffizienten des Diffusionsmodells des Edelgases, so dass das Diffusionsmodell des Edelgases mit den Messungen kohärent ist, und Ableiten eines Verhältnisses zwischen dem Diffusionskoeffizienten und dem veränderten Diffusionskoeffizienten; und

f) Anwenden des Verhältnisses am Diffusionsmodell des wiederzugewinnenden Kohlenwasserstoffes, auf dessen Basis die Menge des wiederzugewinnenden Kohlenwasserstoffes, der zum Zeitpunkt t in der Überwachungszone vorhanden ist, bestimmt wird.

2. Verfahren nach Anspruch 1, bei dem die Überwachungszone ein Grundwasserleiter ist.

3. Verfahren nach einem der vorhergehenden Ansprüche, bei dem ein Austritt des wiederzugewinnenden Kohlenwasserstoffes aus der unterirdischen Formation mit Hilfe der bestimmten Menge an wiederzugewinnendem Kohlenwasserstoff, der in der Überwachungszone vorhanden ist, erfasst wird.

4. Verfahren nach einem der vorhergehenden Ansprüche, bei dem die Frakturierung durch Injektion von Gas, wie Fluorpropan oder Helium, durchgeführt wird.

5. Verfahren nach Anspruch 4, bei dem die Menge des injizierten Gases, das in der Überwachungszone zum Zeitpunkt t vorhanden ist, mit Hilfe eines Diffusionsmodells des injizierten Gases bestimmt wird, an dem das Verhältnis zwischen dem Diffusionskoeffizienten und dem veränderten Diffusionskoeffizienten angewandt wird.

6. Verfahren nach einem der vorhergehenden Ansprüche, bei dem das injizierte Gas mindestens ein Edelgas umfasst.

7. Verfahren nach einem der vorhergehenden Ansprüche, bei dem die Kohlenwasserstoffe Methan umfassen.

8. Verfahren nach einem der vorhergehenden Ansprüche, bei dem das Edelgas Helium oder Argon ist.

**Claims**

1. A method of recovering unconventional hydrocarbons in an underground formation, wherein said unconventional hydrocarbons are recovered by fracturing in said underground formation, at least one noble gas being present in said underground formation, wherein said underground formation is monitored by carrying out the following steps:

a) selecting a diffusion model for said noble gas and a diffusion model for a hydrocarbon to be recovered, each model describing the concentration evolution as a function of time, of depth and of a diffusion coefficient,

b) prior to said hydrocarbon recovery, taking at least a first sample of a fluid present in a zone referred to as subsoil monitoring zone located above said underground formation, and measuring the composition of at least one noble gas within said first sample,

c) during and/or after said hydrocarbon recovery, taking at least a second sample of a fluid present in said monitoring zone, and measuring a concentration of said noble gas within said second sample,

d) repeating step c) at different times,

e) when said concentration of said noble gas increases, modifying said effective diffusion coefficient of the noble gas diffusion model, so that the noble gas diffusion model is coherent with said measurements, and deducing a ratio between said diffusion coefficient and said modified diffusion coefficient, and

f) applying said ratio to the diffusion model of said hydrocarbon to be recovered, from which the amount of said hydrocarbon to be recovered present in said monitoring zone at the time t is determined.

2. A method as claimed in claim 1, wherein said monitoring zone is an aquifer.

3. A method as claimed in any one of the previous claims, wherein leakage of said hydrocarbon to be recovered from the underground formation is detected by means of said determined amount of hydrocarbon to be recovered present in said monitoring zone.

4. A method as claimed in any one of the previous claims, wherein said fracturing is performed by injecting gas such as fluoropropane or helium.

5. A method as claimed in claim 4, wherein the amount of said injected gas present in said monitoring zone at the time t is determined by means of a diffusion model of said injected gas, to which said ratio between said diffusion coefficient and said modified diffusion coefficient is applied.

6. A method as claimed in any one of the previous claims, wherein said injected gas comprises at least one noble gas.

7. A method as claimed in any one of the previous claims, wherein the hydrocarbons contain methane.

8. A method as claimed in any one of the previous claims, wherein said noble gas is helium or argon.

**Fig. 1**

**Fig. 2**

C/Co

Ar

He

CH₄

FP

t

**Fig. 3**

C/Co

He

SCH₄

CH₄

SHe

DEL

TDHe    TDCH₄    t

**Fig. 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2500513 A
- WO 03010534 A